# EUROPEAN PATENT SPECIFICATION

(11) **EP 2 323 642 B1**
(45) Date of publication and mention of the grant of the patent: **19.02.2014**
(21) Application number: 09748494.3
(22) Date of filing: 27.08.2009
(51) Int. Cl.: A61K 31/14, A61K 31/19, A61K 31/195, A61K 31/352, A61K 31/473, A61K 31/49, A61K 31/5415, A61K 31/56, A61K 31/60, A61P 27/02

(54) **Use of EDTA+tromethamine or of photoenhancers, associated to riboflavin for corneal cross-linking in the treatment of the keratoconus or of other corneal ectasic disorders**
Verwendung von EDTA+Tromethamine oder von Photoverstärkungsmitteln, im Zusammenhang mit Riboflavin, zur Hornhautvernetzung bei der Behandlung von Keratokonus oder anderen ektatischen Hornhauterkrankungen
Utilisation de EDTA+tromethamine ou de photoactivateurs, associés à la riboflavine, pour la réticulation cornéenne dans le traitement du kératocône ou d'autres maladies ectasiques de la cornée

(30) Priority: 28.08.2008 IT RM20080472
(43) Date of publication of application: 25.05.2011
(73) Proprietor: Sooft Italia S.p.A., 63833 Montegiorgio (FM) (IT)
(72) Inventor: STAGNI, Marcello, I-95127 Catania (IT); STAGNI, Edoardo, I-95127 Catania (IT); FILIPPELLO, Massimo, I-95129 Catania (IT); CAVALLO, Giovanni, I-00122 Roma (IT); SODO, Eugenio, I-00125 Roma (IT)
(74) Representative: Sarpi, Maurizio
(86) International application number: PCT/IT2009/000392
(87) International publication number: WO 2010/023705

(56) References cited:
- WO-A-2004/058289
- US-A1- 2006 222 597
- WOLLENSAK GREGOR ET AL: "Collagen crosslinking of human and porcine sclera" JOURNAL CATARACT AND REFRACTIVE SURGERY, SURGERY, FAIRFAX, VA, vol. 30, no. 3, 2004, pages 689-695, XP002450030 ISSN: 0886-3350
- SPOERL E, SEILER T.: "Techniques for stiffening the cornea" J REFRACT SURG. 1999 NOV-DEC;15(6):711-3, 1999, XP008107084 University Eye Clinic, Dresden, Germany.
- SPOERL: "Induction of cross-linking in..." EXP EYE RES, no. 66, 1998, pages 97-103, XP008107083

## Description

The present invention relates to the use of enhancers of absorption for the preparation of ophthalmic compositions (in particular collyriums) possibly containing riboflavin, designed to imbibe the corneal stroma without having to proceed, in order to obtain said imbibition, to the removal of the corneal epithelium (de-epithelization) in the practice of the treatment of keratoconus, or other ectasic corneal disorders, by means of cross-linking, and also relates to the corresponding ophthalmic compositions for the technique of corneal cross-linking.

Keratoconus is a serious disease of the cornea since it is a non-inflammatory progressive dystrophy that each year affects approximately 50 persons in every 100,000, generally young people of between 10 and 20 years of age. Keratoconus is a genetic disease with a higher frequency amongst females and at times appears to be correlated to dysfunctions of endocrine glands (hypophysis and thyroid). It can affect both eyes in approximately 85% of cases and has an evolution that may vary from subject to subject.

Upon onset of this disease, there appears an irregular curvature that modifies the refractive power of the cornea, producing distorsions of images and a confused close and distant vision. The patient complains in any case of a reduction of vision, above all distant vision. The vision continues to regress irreversibly, with a consequent need for frequent change of spectacles, and for this reason it may at first be mistaken for a myopia associated to astigmatism.

On account of the congenital structural weakness of the corneal stroma due to said disease, after some years the cornea progressively tends to wear out and thin out towards the apex. There then occurs an irregular curvature of the cornea, which loses its spherical shape and assumes the characteristic cone shape (keratoconus).

Using the biomicroscope there may be noted a considerable reduction in the corneal thickness at the top of keratoconus. Over time, the top of keratoconus becomes opaque on account of an alteration in the nutriment of that part of the cornea, which in the most acute forms can present a corneal curvature of more than 62D and reach a corneal thickness of even 446 µm.

If the disease is neglected, the top can ulcerate with consequent perforation of the cornea; there appear pain, lacrimation and spasm of the eyelids. These changes of the cornea due to keratoconus produce an alteration in the disposition of the corneal protein, causing micro-scars that further distort the images and in some cases prevent passage of light, thus giving rise to a troublesome dazzling feeling, above all at times of the day when the Sun is low on the horizon (sunrise and sunset).

As already mentioned, in order to correct the visus it becomes necessary to change spectacles frequently. Only after the use of spectacles has proven unsatisfactory, in milder forms rigid contact lenses may be applied.

The real problem arises when the cornea affected by keratoconus undergoes considerable thinning or if cicatrization occurs following upon lacerations of the corneal surface, rendering necessary even surgical transplantation of the cornea (keratoplasty).

In 2002 so-called lamellar keratoplasty was introduced in Italy for the treatment of keratoconus, whereby, in practice, not the entire cornea is replaced, but only the outer thickness, i.e., the part affected by the disease.

However, already by 1997 in Germany, in the ophthalmic clinic of the Carl Gustaw Carus University of Dresda, a new safer and less invasive technique was developed, referred to as "corneal cross-linking", which uses in particular riboflavin, activated by a UV laser; in 2005 this technique was tested also in Italy and is by now widely used successfully in various Italian eye clinics.

Corneal cross-linking is a low-invasive method, which uses riboflavin activated by a UV laser (366 nm); said method is painless and is carried out in day-hospital. Cross-linking enables reinforcement of the structure of the cornea affected by keratoconus through the interweaving and increase in links (cross-linking) between the fibres of the corneal collagen. From the clinical studies conducted, this method has proved able to reduce the astigmatism associated to keratoconus as well as to slow down or arrest evolution of keratoconus, thus avoiding the need for transplantation of the cornea. Also other disorders characterized by corneal ecstasia benefit from treatment using the cross-linking method.

Corneal cross-linking is carried out by applying a local corneal anaesthesia for making the abrasion of the corneal epithelium (de-epithelization) having a diameter of 8-9 mm. This is followed by a frequent instillation of a 0.1% riboflavin-based ophthalmic solution followed, after 15 minutes, by irradiation with ultraviolet (UV-A) emitter for 5 minutes, and then by new instillation and new irradiation for a total of 6 cycles (30 minutes in all).

Riboflavin, in particular riboflavin phosphate, which is commonly used in corneal cross-linking, is a hydrophilic photosensitizing and photopolymerizing molecule with a poor capacity for diffusing through the epithelium and hence reaching the corneal stroma. It is therefore necessary to facilitate absorption thereof and complete imbibition of the corneal stroma before starting the irradiation with UV-A, by means of removal of the corneal epithelium (de-epithelization). This procedure can create, albeit rarely, complications at a corneal level, pain, in addition to being a method that renders the task of the oculist more difficult.

It would hence be desirable to improve the absorption of riboflavin, without having to resort to de-epithelization of the cornea, hence obtaining a non-invasive corneal cross-linking with elimination or reduction of the anaesthesia and consequent fast healing without pain or possible complications.

It would moreover be desirable to envisage compounds that enable ease of epithelial absorption but are provided with equal or higher activity than riboflavin in determining photosensitization and photopolymerization of the collagen fibrils; said compounds should preferably be activated at intensities of light close to the visible in order to overcome the harmful effects of the repeated cycles of UV radiation necessary for activation of riboflavin.

It has now been surprisingly discovered that by administering on the cornea ophthalmic compositions containing enhancers chosen from among:
bio-enhancers (i.e., substances that favour the passage of riboflavin or of other photosensitizing and photopolymerizing substances through the corneal epithelium, enabling absorption by the corneal stroma itself), namely EDTA associated to tromethamine, and/or
photo-enhancers (i.e., photosensitive and photopolymerizing substances that can be readily absorbed by the epithelium and that, like riboflavin, can also be activated by light to form corneal cross-linking), namely the dyes acridine yellow, quinidine yellow, methylene blue, and erythrosine, the structures of which are given below:
   acridine yellow:
      useful absorption: 460 nm 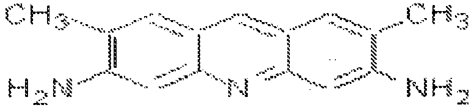
      quinidine yellow 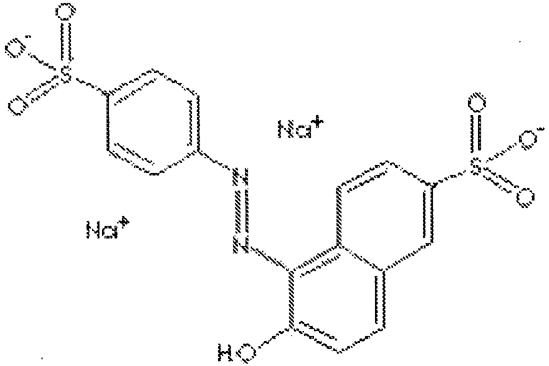
      methylene blue
         useful absorption: 668 nm 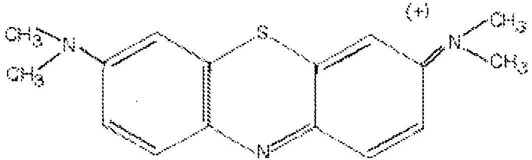
      erythrosine B
         2',4',5',7'-Tetraiodofluorescein disodium salt Absorption: 525 nm 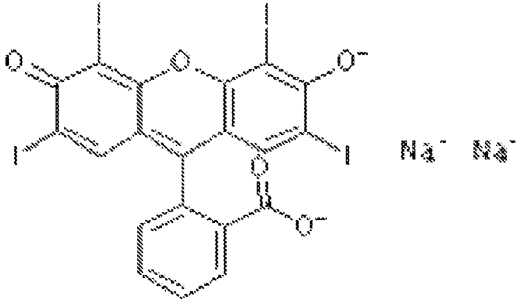
      Molecular formula:
         C₂₀H₆I₄Na₂O₅
      Molecular weight:
         879.86
associated to riboflavin, an improved corneal cross-linking is obtained by means of an irradiation close to the visible that is less harmful, without any need for de-epithelization.

The present invention hence envisages the use of the enhancers described above, either alone or variously mixed together, with riboflavin, for the preparation of ophthalmic compositions to be used for cross-linking in keratoconus or in other corneal ectasias.

The enhancers according to the present invention can be used for application directly on the cornea for cross-linking of keratoconus or other corneal ectasias without proving cytotoxic.

Forming a further subject of the present invention are ophthalmic compositions containing the enhancers mentioned above possibly associated to riboflavin; said ophthalmic composition can be prepared according to known techniques and can contain in particular:
- one or more bio-enhancers with one or more photo-enhancers and possibly riboflavin;
- one or more bio-enhancers with possibly riboflavin;
- one or more photo-enhancers with possibly riboflavin.

After ocular administration of the compositions described above containing just one or more bio-enhancers or else one or more photo-enhancers, there may possibly be applied directly on the cornea a solution of one or more photosensitizing and photopolymerizing substances, in particular riboflavin.

The bio-enhancers of the present invention are present in appropriate amounts in all the compositions described above chosen between 0.001 wt% and 5 wt% with respect to the composition.

In particular the enhancer capability of the present formulation mainly depends on the EDTA and tromethamine association since the two compounds form together a ion-pair between the EDTA non salified carboxyl and the tromethamine which has a notable membrane penetrative capacity.

Experimental data have pointed out that, if the tromethamine is preferably present at between 0.05 wt% and 0.5 wt%, and EDTA is preferably present at between 0.05 wt% and 0.5 wt%, the riboflavin absorption is immediate.

The photosensitizing and photopolymerizing substances (photo-enhancers), amongst which riboflavin, of the present invention are used in appropriate amounts chosen between 0.001 wt% and 1 wt% with respect to the composition.

In addition, the riboflavin preferably used in the present invention is riboflavin phosphate in appropriate amounts in all the compositions described above; in particular it is preferably present at between 0.05 wt% and 0.3 wt% of the composition of the present invention.

The ophthalmic compositions of the present invention can be prepared in the technical form of collyriums, eye-drops, eye-washes, ointments, and in any case in all the pharmaceutical technical forms that enable a corneal application according to known techniques; given hereinafter are examples provided by way of illustration.
Formulations are reported below, the dosage of the individual components is expressed in weight percentage.

### EXAMPLE 1

| | |
|---|---|
| PERMEABILIZING SOLUTION | 3 |
| Dextran T500 | |
| EDTA 0.1% Polysorbate 80 1% | |
| Tromethamine 0.1% | |

| Ingredients | % w/w |
|---|---|
| DextranT500 | 15 |
| Sodium EDTA | 0.1 |
| Polysorbate 80 1% | 1 |
| Tromethamine | 0.1 |
| Dihydrated monobasic sodium phosphate | 0.067 |
| Dihydrated dibasic sodium phosphate | 0.285 |
| NaCl | 0.00 |
| Distilled water | Up to 100 g |
| Isotonicity: hypotonic | 142MOs |
| PH | 7.2 |
| Sterilized filtration | Yes |

### EXAMPLE 2

| | |
|---|---|
| PERMEABILIZING SOLUTION | 4 |
| Dextran T500 | |
| EDTA 0.1% Tromethamine 0.1 | |

| Ingredients | % w/w |
|---|---|
| Riboflavin phosphate dihydrated sodium salt | 0.147 |
| DextranT500 | 15 |
| Sodium EDTA | 0.1 |
| Tromethamine | 0.1 |
| Dihydrated monobasic sodium phosphate | 0.067 |
| Dihydrated dibasic sodium phosphate | 0.285 |
| NaCl | 0.00 |
| Distilled water | Up to 100 g |
| Isotonicity: hypotonic | 142MOs |
| PH | 7.2 |
| Sterilized filtration | Yes |

### EXAMPLE 3

| | |
|---|---|
| PERMEABILIZING SOLUTION | 5 |
| Dextran T500 | |
| EDTA 0.1% Tromethamine 0.1 | |

| Ingredients | % w/w |
|---|---|
| DextranT500 | 15 |
| Sodium EDTA | 0.2 |
| Tromethamine | 0.2 |
| Dihydrated monobasic sodium phosphate | 0.067 |
| Dihydrated dibasic sodium phosphate | 0.285 |
| NaCl | 0.00 |
| Distilled water | Up to 100 g |
| Isotonicity: hypotonic | 142MOs |
| PH | 7.2 |
| Sterilized filtration | Yes |

Note that, when the concentration of tromethamine is increased, it is necessary to increase NaH₂PO₄ sufficiently so as to guarantee a pH of 7-7.2.

### EXAMPLE 4

| | |
|---|---|
| Preparation | E |
| RIBOFLAVIN PHOSPHATE 0.147w/w | |
| Dextran T500 | |
| Quinoline Yellow 0.050% | |

| Ingredients | % w/w |
|---|---|
| Riboflavin phosphate dihydrated sodium salt | 0.147 |
| DextranT500 | 20 |
| Quinoline Yellow | 0.050 |
| Dihydrated monobasic sodium phosphate | 0.067 |
| Dihydrated dibasic sodium phosphate | 0.285 |
| NaCl | 0.00 |
| Distilled water | Up to 100 g |
| Isotonicity: hypotonic | 139 mMOs |
| PH | 7.2 |
| Sterilized filtration | Yes |

### EXAMPLE 5

| | |
|---|---|
| Preparation HYPOTONIC | F |
| RIBOFLAVIN PHOSPHATE 0.147 w/w | |
| Dextran T500 | |
| Acridine Yellow 0.050% | |

| Ingredients | % w/w |
|---|---|
| Riboflavin phosphate dihydrated sodium salt | 0.147 |
| DextranT500 | 20 |
| Acridine Yellow | 0.050 |
| Dihydrated monobasic sodium phosphate | 0.067 |
| Dihydrated dibasic sodium phosphate | 0.285 |
| NaCl | 0.00 |
| Distilled water | Up to 100 g |
| Isotonicity: hypotonic | 139 mMOs |
| PH | 7.1 |
| Sterilized filtration | Yes |

### EXAMPLE 6

| | |
|---|---|
| Preparation HYPOTONIC | G |
| RIBOFLAVIN PHOSPHATE 0.147 w/w | |
| Dextran T500 | |
| ErytrosinB 0.050% | |

| Ingredients | % w/w |
|---|---|
| Riboflavin phosphate dihydrated sodium salt | 0.147 |
| DextranT500 | 20 |
| Erythrosin B | 0.050 |
| Dihydrated monobasic sodium phosphate | 0.067 |
| Dihydrated dibasic sodium phosphate | 0.285 |
| NaCl | 0.00 |
| Distilled water | Up to 100 g |
| Isotonicity: hypotonic | 139 mMOs |
| PH | 7.1 |
| Sterilized filtration | Yes |

### EXAMPLE 7

| | |
|---|---|
| Preparation | H |
| RIBOFLAVIN PHOSPHATE 0.147w/w | |
| Dextran T500 | |
| Methylene blue 0.050% | |

| Ingredients | % w/w |
|---|---|
| Riboflavin phosphate dihydrated sodium salt | 0.147 |
| DextranT500 | 20 |
| Methylene blue | 0.050 |
| Dihydrated monobasic sodium phosphate | 0.067 |
| Dihydrated dibasic sodium phosphate | 0.285 |
| NaCl | 0.00 |
| Distilled water | Up to 100 g |
| Isotonicity: hypotonic | 139 mMOs |
| PH | 7.1 |
| Sterilized filtration | Yes |

It should be pointed out that the formulations 1-7 may also be isotonic or even hypertonic with the addition of sufficient sodium chloride (or other osmolyte) to achieve said purpose.

## Claims

1. Use of EDTA associated to tromethamine, and/or one or more photoenhancers chosen among: acridine yellow, quinidine yellow, methylene blue, erithrosine, for the preparation of ophthalmic compositions for the method of corneal cross-linking in the treatment of the keratoconus or of other corneal ectasic disorders, wherein the composition comprises riboflavin or riboflavin phosphate.

2. Use according to Claim 1 wherein the bio-enhancers are present in an amount chosen between 0.0001-5 wt% with respect to the composition, in particular EDTA is between 0.001 and 1 wt%, tromethamine is between 0.001 and 2 wt%.

3. Use according to Claim 2, wherein EDTA is present in an amount between 0.05 and 0.5 wt% and tromethamine in an amount between 0.05 and 0.5 wt%.

4. Use according to any one of Claims 1 to 3, wherein the photo-enhancers are present in an amount of between 0.001 and 1 wt%, and riboflavin phosphate is present in an amount of between 0.05 and 0.3 wt%.

5. Use according to any one of Claims 1 to 4, wherein riboflavin phosphate is present in an amount of between 0.05 and 0.3 wt%.

6. Ophthalmic composition comprising EDTA associated to tromethamine, and/or one or more photoenhancers chosen among: acridine yellow, quinidine yellow, methylene blue, erithrosine, for use in the method of corneal cross-linking in the treatment of the keratoconus or of other corneal ectasic disorders, wherein EDTA associated to tromethamine is present with one or more photo-enhancers and riboflavin, or
wherein EDTA associated to tromethamine is present with riboflavin, or
wherein one or more photo-enhancers are present with riboflavin.

7. Composition for use according to Claim 6 wherein the bio-enhancers are present in an amount chosen between 0.0001-5 wt% with respect to the composition, in particular EDTA is between 0.001 and 1 wt%, tromethamine is between 0.001 and 2 wt%.

8. Composition for use according to Claim 7, wherein EDTA is present in an amount between 0.05 and 0.5 wt% and tromethamine in an amount between 0.05 and 0.5 wt%.

9. Composition for use according to any one of Claims 6 to 8, wherein the photo-enhancers are present in an amount of between 0.001 and 1 wt%, and riboflavin phosphate is present in an amount of between 0.05 and 0.3 wt%.

10. Composition for use according to any one of Claims 7 to 9, wherein riboflavin phosphate is present in an amount of between 0.05 and 0.3 wt%.

11. Use according to any of claims 1-5, wherein the ophthalmic composition is a collyrium.

12. Composition for use according to any of claims 6-10, wherein the composition is a collyrium.

## Patentansprüche

1. Verwendung von mit Tromethamin verknüpfter EDTA, und/oder einem oder mehreren Lichtverstärkern, die ausgewählt sind aus: Acridingelb, Chinidingelb, Methylenblau, Erythrosin zur Herstellung von ophthalmischen Zusammensetzungen für das Verfahren einer Hornhautvernetzung bei der Behandlung von Keratokonus (Hornhautkegel) oder anderen Hornhautektasieerkrankungen, wobei die Zusammensetzung Riboflavin oder Riboflavinphosphat enthält.

2. Verwendung nach Anspruch 1, wobei die Bioenhancer in einem Anteil, der zwischen 0,0001 bis 5 Gew.-% bezüglich der Zusammensetzung gewählt ist, vorhanden sind, insbesondere die EDTA zwischen 0,001 und 1 Gew.-% liegt, Tromethamin zwischen 0,001 und 2 Gew.-% liegt.

3. Verwendung nach Anspruch 2, bei der die EDTA in einem Anteil zwischen 0,05 und 0,5 Gew.-% und Tromethamin in einem Anteil von zwischen 0,05 und 0,5 Gew.-% vorhanden ist.

4. Verwendung nach einem der Ansprüche 1 bis 3, wobei die Lichtverstärker in einem Anteil von zwischen 0,001 und 1 Gew.-% vorhanden sind und Riboflavinphosphat in einem Anteil von zwischen 0,05 und 0,3 Gew.-% vorhanden ist.

5. Verwendung nach einem der Ansprüche 1 bis 4, wobei Riboflavinphosphat in einem Anteil von zwischen 0,05 und 0,3 Gew.-% vorhanden ist.

6. Ophthalmische Zusammensetzung, umfassend mit Tromethamin verknüpfte EDTA und/oder einen oder mehrere aus: Acridingelb, Chinidingelb, Methylenblau, Erythrosin ausgewählte Lichtverstärker zur Verwendung in dem Verfahren von Hornhautvernetzung bei der Behandlung von Keratokonus oder anderer Hornhautektasieerkrankungen, wobei mit Tromethamin verknüpfte EDTA mit einem oder mehreren Lichtverstärkern und Riboflavin vorhanden ist, oder wobei mit Tromethamin verknüpfte EDTA mit Riboflavin vorhanden ist, oder wobei ein oder mehrere Lichtverstärker mit Riboflavin vorhanden sind.

7. Zusammensetzung zur Verwendung nach Anspruch 6, bei der die Bioenhancer in einem Anteil vorhanden sind, der zwischen 0,0001 bis 5 Gew.-% bezüglich der Zusammensetzung gewählt ist, die EDTA insbesondere zwischen 0,001 und 1 Gew.-% und Tromethamin zwischen 0,001 und 2 Gew.-% liegt.

8. Zusammensetzung zur Verwendung nach Anspruch 7, bei der die EDTA in einem Anteil zwischen 0,05 und 0,5 Gew.-% und Tromethamin in einem Anteil zwischen 0,05 und 0,5 Gew.-% vorhanden sind.

9. Zusammensetzung zur Verwendung nach einem der Ansprüche 6 bis 8, bei der die Lichtverstärker in einem Anteil von zwischen 0,001 und 1 Gew.-% vorhanden sind und Riboflavinphosphat in einem Anteil von zwischen 0,05 und 0,3 Gew.-% vorhanden ist.

10. Zusammensetzung zur Verwendung nach einem der Ansprüche 7 bis 9, bei der Riboflavinphosphat in einem Anteil von zwischen 0,05 und 0,3 Gew.-% vorhanden sind.

11. Verwendung nach einem der Ansprüche 1 bis 5, wobei die ophthalmische Zusammensetzung ein Augenwasser ist.

12. Zusammensetzung zur Verwendung nach einem der Ansprüche 6 bis 10, wobei die Zusammensetzung ein Augenwasser ist.

## Revendications

1. Utilisation d'EDTA associé à la trométhamine, et/ou d'un ou plusieurs photo-activateurs choisis parmi : jaune d'acridine, jaune de quinidine, bleu de méthylène, érythrosine, pour la préparation de compositions ophtalmiques pour le procédé de réticulation cornéenne dans le traitement du kératocône ou d'autres maladies ectasiques de la cornée, dans laquelle la composition comprend de la riboflavine ou du phosphate de riboflavine.

2. Utilisation selon la revendication 1, dans laquelle les bio-activateurs sont présents dans une quantité choisie entre 0,0001 et 5% en poids par rapport à la composition, en particulier l'EDTA est compris entre 0,001 et 1% en poids, la trométhamine est comprise entre 0,001 et 2% en poids.

3. Utilisation selon la revendication 2, dans laquelle l'EDTA est présent dans une quantité entre 0,05 et 0,5% en poids et la trométhamine dans une quantité entre 0,05 et 0,5% en poids.

4. Utilisation selon l'une quelconque des revendications 1 à 3, dans laquelle les photo-activateurs sont présents dans une quantité comprise entre 0,001 et 1% en poids, et le phosphate de riboflavine est présent dans une quantité comprise entre 0,05 et 0,3% en poids.

5. Utilisation selon l'une quelconque des revendications 1 à 4, dans laquelle le phosphate de riboflavine est présent dans une quantité comprise entre 0,05 et 0,3% en poids.

6. Composition ophtalmique comprenant de l'EDTA associé à la trométhamine, et/ou un ou plusieurs photo-activateurs choisis parmi : jaune d'acridine, jaune de quinidine, bleu de méthylène, érythrosine, pour l'utilisation dans le procédé de réticulation cornéenne dans le traitement du kératocône ou d'autres maladies ectasiques de la cornée, dans laquelle l'EDTA associé à la trométhamine est présent avec un ou plusieurs photo-activateurs et de la riboflavine, ou dans laquelle l'EDTA associé à la trométhamine est présent avec de la riboflavine, ou dans laquelle un ou plusieurs photo-activateurs sont présents avec de la riboflavine.

7. Composition pour l'utilisation selon la revendication 6, dans laquelle les bio-activateurs sont présents dans une quantité choisie entre 0,0001 et 5% en poids par rapport à la composition, en particulier l'EDTA est compris entre 0,001 et 1% en poids, la trométhamine est comprise entre 0,001 et 2% en poids.

8. Composition pour l'utilisation selon la revendication 7, dans laquelle l'EDTA est présent dans une quantité entre 0,05 et 0,5% en poids et la trométhamine dans une quantité entre 0,05 et 0,5% en poids.

9. Composition pour l'utilisation selon l'une quelconque des revendications 6 à 8, dans laquelle les photo-activateurs sont présents dans une quantité comprise entre 0,001 et 1% en poids, et le phosphate de riboflavine est présent dans une quantité comprise entre 0,05 et 0,3% en poids.

10. Composition pour l'utilisation selon l'une quelconque des revendications 7 à 9, dans laquelle le phosphate de riboflavine est présent dans une quantité comprise entre 0,05 et 0,3% en poids.

11. Utilisation selon l'une quelconque des revendications 1 à 5, dans laquelle la composition ophtalmique est un collyre.

12. Composition pour l'utilisation selon l'une quelconque des revendications 6 à 10, dans laquelle la composition est un collyre.
